# EUROPEAN PATENT SPECIFICATION

(11) **EP 4 378 420 B1**
(45) Date of publication and mention of the grant of the patent: **05.03.2025**
(21) Application number: 24161759.6
(22) Date of filing: 23.01.2009
(51) Int. Cl.: A61F 2/24

(54) **STENTS FOR PROSTHETIC HEART VALVES**
STENTS FÜR HERZKLAPPENPROTHESEN
STENTS POUR VALVULES CARDIAQUES PROTHÉTIQUES

(30) Priority: 24.01.2008 US 6220708 P; 26.06.2008 US 7590208 P
(43) Date of publication of application: 05.06.2024
(62) Divisional of application: 18197957.6
(73) Proprietor: Medtronic, Inc., Minneapolis, MN 55432 (US)
(72) Inventor: P. TABOR, Charles, Minneapolis, 55432 (US); ELSA EBERHARDT, Carol, Minneapolis, 55432 (US); G. LASKE, Timothy, Minneapolis, 55432 (US); R. RYAN, Timothy, Minneapolis, 55432 (US); MORROW, Joseph, Minneapolis, 55432 (US); TAM, Tammy, Minneapolis, 55432 (US); CAMPBELL, Louis, Minneapolis, 55432 (US); A. GLYNN, Brian, Minneapolis, 55432 (US); B. RUBIN, Anne, Minneapolis, 55432 (US); J. TUCHEK, Michael, Minneapolis, 55432 (US)
(74) Representative: Zimmermann & Partner Patentanwälte mbB

(56) References cited:
- US-A1- 2005 075 584
- US-A1- 2006 122 692
- US-A1- 2007 100 435
- US-A1- 2008 004 688

## Description

### TECHNICAL FIELD

The present invention relates to prosthetic heart valves. More particularly, disclosed herein are devices, methods, and delivery systems for percutaneously implanting prosthetic heart valves.

### BACKGROUND

Diseased or otherwise deficient heart valves can be repaired or replaced using a variety of different types of heart valve surgeries. Typical heart valve surgeries involve an open-heart surgical procedure that is conducted under general anesthesia, during which the heart is stopped while blood flow is controlled by a heart-lung bypass machine. This type of valve surgery is highly invasive and exposes the patient to a number of potentially serious risks, such as infection, stroke, renal failure, and adverse effects associated with use of the heart-lung machine, for example.

Recently, there has been increasing interest in minimally invasive and percutaneous replacement of cardiac valves. Such surgical techniques involve making a very small opening in the skin of the patient into which a valve assembly is inserted in the body and delivered to the heart via a delivery device similar to a catheter. This technique is often preferable to more invasive forms of surgery, such as the open-heart surgical procedure described above. In the context of pulmonary valve replacement, U.S. Patent Application Publication Nos. 2003/0199971 A1 and 2003/0199963 A1, both filed by Tower, et al., describe a valved segment of bovine jugular vein, mounted within an expandable stent, for use as a replacement pulmonary valve. The replacement valve is mounted on a balloon catheter and delivered percutaneously via the vascular system to the location of the failed pulmonary valve and expanded by the balloon to compress the valve leaflets against the right ventricular outflow tract, anchoring and sealing the replacement valve. As described in the articles: "Percutaneous Insertion of the Pulmonary Valve", Bonhoeffer, et al., Journal of the American College of Cardiology 2002; 39: 1664 - 1669 and "Transcatheter Replacement of a Bovine Valve in Pulmonary Position", Bonhoeffer, et al., Circulation 2000; 102: 813 - 816, the replacement pulmonary valve may be implanted to replace native pulmonary valves or prosthetic pulmonary valves located in valved conduits.

Various types and configurations of prosthetic heart valves are used in percutaneous valve procedures to replace diseased natural human heart valves. The actual shape and configuration of any particular prosthetic heart valve is dependent to some extent upon the valve being replaced (i.e., mitral valve, tricuspid valve, aortic valve, or pulmonary valve). In general, the prosthetic heart valve designs attempt to replicate the function of the valve being replaced and thus will include valve leaflet-like structures used with either bioprostheses or mechanical heart valve prostheses. In other words, the replacement valves may include a valved vein segment that is mounted in some manner within an expandable stent to make a stented valve. In order to prepare such a valve for percutaneous implantation, the stented valve can be initially provided in an expanded or uncrimped condition, then crimped or compressed around the balloon portion of a catheter until it is as close to the diameter of the catheter as possible.

Other percutaneously-delivered prosthetic heart valves have been suggested having a generally similar configuration, such as by Bonhoeffer, P. et al., "Transcatheter Implantation of a Bovine Valve in Pulmonary Position." Circulation, 2002; 102:813-816, and by Cribier, A. et al. "Percutaneous \Transcatheter Implantation of an Aortic Valve Prosthesis for Calcific Aortic Stenosis." Circulation, 2002; 106:3006-3008. These techniques rely at least partially upon a frictional type of engagement between the expanded support structure and the native tissue to maintain a position of the delivered prosthesis, although the stents can also become at least partially embedded in the surrounding tissue in response to the radial force provided by the stent and balloons used to expand the stent. Thus, with these transcatheter techniques, conventional sewing of the prosthetic heart valve to the patient's native tissue is not necessary. Similarly, in an article by Bonhoeffer, P. et al. titled "Percutaneous Insertion of the Pulmonary Valve." J Am Coll Cardiol, 2002; 39:1664-1669, percutaneous delivery of a biological valve is described. The valve is sutured to an expandable stent within a previously implanted valved or non-valved conduit, or a previously implanted valve. Again, radial expansion of the secondary valve stent is used for placing and maintaining the replacement valve.
Document US 2005/075584 A1 relates to a minimally invasive valve replacement system.
Document US 2008/004688 A1 relates to an implantable prosthetic valve.

Although there have been advances in percutaneous valve replacement techniques and devices, there is a continued desire to provide different designs of cardiac valves that can be implanted in a minimally invasive and percutaneous manner. It is additionally desirable to provide valves that are resistant to migration after they are implanted.

### SUMMARY The invention is defined in the appended claims.

The replacement heart valves of the invention each include a stent to which a valve structure is attached. The stents of the invention include a wide variety of structures and features that can be used alone or in combination with features of other stents of the invention. Many of the structures are compressible to a relatively small diameter for percutaneous delivery to the heart of the patient, and then are expandable either via removal of external compressive forces (e.g., self-expanding stents), or through application of an outward radial force (e.g., balloon expandable stents). The devices delivered by the delivery systems described herein can be used to deliver stents, valved stents, or other interventional devices such as ASD (atrial septal defect) closure devices, VSD (ventricular septal defect) closure devices, or PFO (patent foramen ovale) occluders.

Methods for insertion of the replacement heart valves of the invention include delivery systems that can maintain the stent structures in their compressed state during their insertion and allow or cause the stent structures to expand once they are in their desired location. In addition, delivery methods of the invention can include features that allow the stents to be retrieved for removal or relocation thereof after they have been deployed or partially deployed from the stent delivery systems. The methods may include implantation of the stent structures using either an antegrade or retrograde approach. Further, in many of the delivery approaches of the invention, the stent structure is rotatable in vivo to allow the stent structure to be positioned in a desired orientation.

The stent structures of the invention can provide resistance to leaflet abrasion via the configuration of the wires or other structural elements relative to each other. Other stent structures can provide for reduced crown density and various other configurations of wire shapes and features for use with attached valves for valve replacement procedures.

### BRIEF DESCRIPTION OF THE DRAWINGS

The present invention will be further explained with reference to the appended Figures, wherein like structure is referred to by like numerals throughout the several views, and wherein:
Figure 1 is a front view of an embodiment of a stent in accordance with the invention;
Figure 2 is a front view of an embodiment of a stent in accordance with the invention;
Figure 3 is a front view of an embodiment of a stent in accordance with the invention;
Figure 4 is a perspective view of a stent embodiment in accordance with the invention;
Figure 5 is a top view of another stent embodiment;
Figure 6 is a front view of another stent embodiment;
Figure 7 is a front view of another stent embodiment;
Figure 8 is a perspective view of another stent embodiment;
Figure 9 is a perspective view of a stent embodiment having extending elements and positioned on a mandrel;
Figure 10 is a front view of an exemplary delivery system that can be used for delivering a stent of the type illustrated in Figure 9;
Figure 11-13 are enlarged front views of a portion of a delivery system for delivering a stent of the type shown in Figure 9, including three sequential delivery steps;
Figure 14 is a front schematic view of a stent positioned in an aorta;
Figures 15-18 are perspective views of different stent embodiments, each positioned within a heart vessel;
Figure 19 is a front view of a stent embodiment;
Figure 20 is a front view of a stent embodiment;
Figure 21 is a top view of the stent of Figure 20;
Figure 22 is a schematic front view of the stent of Figure 20 positioned in a heart vessel;
Figure 23 is a front view of another stent embodiment;
Figure 24 is a side view of the stent of Figure 23;
Figure 25 is a perspective view of the stent of Figure 23, positioned on a mandrel;
Figure 26 is a top view of the stent of Figure 23 positioned relative to a schematic view of a heart vessel, wherein the stent includes leaflets in its interior portion;
Figure 27 is a top view of the stent of Figure 23;
Figure 28 is a perspective top view of the stent of Figure 23 positioned in a heart;
Figure 29 is a front view of another embodiment of a stent positioned on a mandrel;
Figures 30 and 31 are front and perspective views respectively, of a solid model of a stent of the type illustrated in Figure 29;
Figures 32 and 33 are front perspective views, respectively, of a stent embodiment;
Figures 34 and 35 are front views of a valved stent of the invention;
Figure 36 is a schematic front view of a stent assembly being delivered to a heart valve;
Figure 37 is a front view of a stent assembly positioned in a heart valve;
Figure 38 is a front view of the stent assembly shown in Figures 36 and 37;
Figure 39 is a front view of a stent assembly having a length L positioned in a heart vessel;
Figure 40 is a top view of another stent embodiment positioned relative to a schematic view of an anatomical position in a heart;
Figure 41 is a top view of another stent embodiment;
Figure 42 is a top view of another stent positioned relative to the interventricular septum and the mitral apparatus;
Figures 43-45 are perspective views of additional stent embodiments;
Figure 46 is a front view of another stent embodiment;
Figures 47-50 are front schematic views of embodiments of stents positioned in a heart vessel;
Figures 51-53 are front views of a different stents positioned relative to a portion of a heart valve that is cut-away for clarity; and
Figure 54 is a top cross-sectional view of a valve attached within a stent frame.

### DETAILED DESCRIPTION

As referred to herein, the prosthetic heart valves used in accordance with various devices and methods of heart valve delivery may include a wide variety of different configurations, such as a prosthetic heart valve having tissue leaflets or a synthetic heart valve having polymeric, metallic, or tissue-engineered leaflets, and can be specifically configured for replacing any heart valve. That is, while much of the description herein refers to replacement of aortic valves, the prosthetic heart valves of the invention can also generally be used for replacement of native mitral, pulmonic, or tricuspid valves, for use as a venous valve, or to replace a failed bioprosthesis, such as in the area of an aortic valve or mitral valve, for example.

Although each of the valves used with the delivery devices and methods described herein would typically include leaflets attached within an interior area of a stent, the leaflets are not shown in many of the illustrated embodiments for clarity purposes. According to the invention, the stents described herein include a support structure comprising a number of strut or wire portions arranged relative to each other to provide a desired compressibility, strength, and leaflet attachment zone(s) to the heart valve. Other details on particular configurations of the stents of the invention are also described below; however, in general terms, stents of the invention are generally tubular support structures, and leaflets will be secured to the support structure to provide a valved stent. The leaflets can be formed from a variety of materials, such as autologous tissue, xenograph material, or synthetics as are known in the art. The leaflets may be provided as a homogenous, biological valve structure, such as a porcine, bovine, or equine valve. Alternatively, the leaflets can be provided independent of one another (e.g., bovine or equine pericardial leaflets) and subsequently assembled to the support structure of the stent. In another alternative, the stent and leaflets can be fabricated at the same time, such as may be accomplished using high strength nano-manufactured NiTi films of the type produced at Advanced Bio Prosthetic Surfaces Ltd. (ABPS) of San Antonio, Texas, for example. The support structures are generally configured to accommodate three leaflets; however, the replacement prosthetic heart valves of the invention can incorporate more or less than three leaflets.

In more general terms, the combination of a support structure with one or more leaflets can assume a variety of other configurations that differ from those shown and described, including any known prosthetic heart valve design. In certain embodiments of the invention, the support structure with leaflets utilize certain features of known expandable prosthetic heart valve configurations, whether balloon expandable, self-expanding, or unfurling (as described, for example, in U.S. Patent Nos. 3,671,979; 4,056,854; 4,994,077; 5,332,402; 5,370,685; 5,397,351; 5,554,185; 5,855,601; and 6,168,614; U.S. Patent Application Publication No. 2004/0034411; Bonhoeffer P., et al., "Percutaneous Insertion of the Pulmonary Valve", Pediatric Cardiology, 2002; 39:1664-1669; Anderson H R, et al., "Transluminal Implantation of Artificial Heart Valves", EUR Heart J., 1992; 13:704-708; Anderson, J. R., et al., "Transluminal Catheter Implantation of New Expandable Artificial Cardiac Valve", EUR Heart J., 1990, 11: (Suppl) 224a; Hilbert S. L., "Evaluation of Explanted Polyurethane Trileaflet Cardiac Valve Prosthesis", J Thorac Cardiovascular Surgery, 1989; 94:419-29; Block P C, "Clinical and Hemodyamic Follow-Up After Percutaneous Aortic Valvuloplasty in the Elderly", The American Journal of Cardiology, Vol. 62, Oct. 1, 1998; Boudjemline, Y., "Steps Toward Percutaneous Aortic Valve Replacement", Circulation, 2002; 105:775-558; Bonhoeffer, P., "Transcatheter Implantation of a Bovine Valve in Pulmonary Position, a Lamb Study", Circulation, 2000:102:813-816; Boudjemline, Y., "Percutaneous Implantation of a Valve in the Descending Aorta In Lambs", EUR Heart J, 2002; 23:1045-1049; Kulkinski, D., "Future Horizons in Surgical Aortic Valve Replacement: Lessons Learned During the Early Stages of Developing a Transluminal Implantation Technique", ASAIO J, 2004; 50:364-68;

Orientation and positioning of the stents of the invention may be accomplished either by self-orientation of the stents (such as by interference between features of the stent and a previously implanted stent or valve structure) or by manual orientation of the stent to align its features with anatomical or previous bioprosthetic features, such as can be accomplished using fluoroscopic visualization techniques, for example. For example, when aligning the stents of the invention with native anatomical structures, they should be aligned so as to not block the coronary arteries, and native mitral or tricuspid valves should be aligned relative to the anterior leaflet and/or the trigones/commissures.

Some embodiments of the support structures of the stents described herein can be a series of wires or wire segments arranged so that they are capable of transitioning from a collapsed state to an expanded state. In some embodiments, a number of individual wires comprising the support structure can be formed of a metal or other material. These wires are arranged in such a way that a support structure allows for folding or compressing to a contracted state in which its internal diameter is greatly reduced from its internal diameter in an expanded state. In its collapsed state, such a support structure with attached valves can be mounted over a delivery device, such as a balloon catheter, for example. The support structure is configured so that it can be changed to its expanded state when desired, such as by the expansion of a balloon catheter. The delivery systems used for such a stent should be provided with degrees of rotational and axial orientation capabilities in order to properly position the new stent at its desired location.

The wires of the support structure of the stents in other embodiments can alternatively be formed from a shape memory material such as a nickel titanium alloy (e.g., Nitinol) or a very high-tensile material that will expand to its original state after compression and removal of external forces. With this material, the support structure is self-expandable from a contracted state to an expanded state, such as by the application of heat, energy, and the like, or by the removal of external forces (e.g., compressive forces). This support structure can be repeatedly compressed and re-expanded without damaging the structure of the stent. In addition, the support structure of such an embodiment may be laser cut from a single piece of material or may be assembled from a number of different components. For these types of stent structures, one example of a delivery system that can be used includes a catheter with a retractable sheath that covers the stent until it is to be deployed, at which point the sheath can be retracted to allow the stent to expand. Alternatively, the stent structures of the invention can be implanted using conventional surgical techniques and/or minimally invasive surgical procedures. In such cases, the stents of the invention can advantageously require relatively few or no sutures to secure the stent to an anatomical location within the patient.

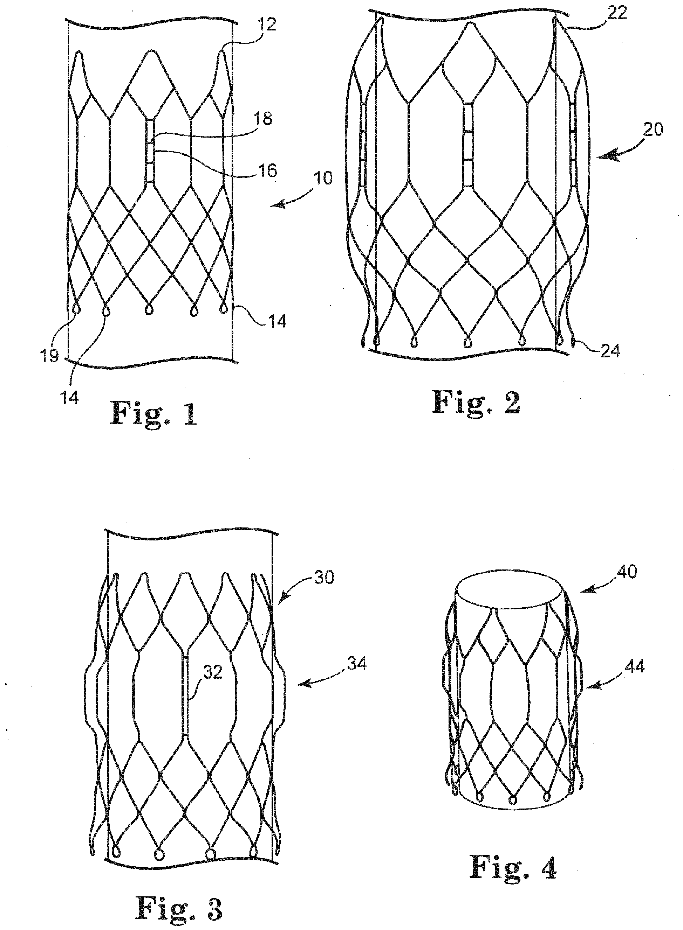

Referring now to the Figures, wherein the components are labeled with like numerals throughout the several Figures, and initially to Figures 1-5 illustrate stents 10, 20, 30, and 40, respectively, each of which is positioned over a mandrel. With particular reference to Figure 1, stent 10 includes a first end 12 having six crowns and a second end 14 having twelve crowns. Each of the stent crowns at the second end 14 includes a loop or eyelet 19 that can be used for attachment to a delivery system and/or tissue valve, for example. It is contemplated that each of the crowns at the second end includes a loop or eyelet 19, as shown, or that only some of the crowns include such a loop or eyelet. The size and shape of the loops 19 can all be the same on a single stent, or they can have different sizes and/or shapes. Stent 10 further includes at least one longitudinal post 16, which can be used for attachment of tissue to the stent, along with providing additional stability to the first end 12 of the stent. The longitudinal post 16 extends generally along the annular region of the stent 10 and has a height that accommodates attachment of leaflet material. That is, the height of the post 16 is generally the same as the desired commissural height for the stent 10. According to the invention, the longitudinal posts 16 are comprised of two bars or vertical portions that are spaced from each other by a sufficient distance to allow leaflets to be drawn between the vertical portions at the leaflet commissures. Other skirt material portions and/or commissure protection features can also be drawn through the space between the vertical portions. The space between the vertical portions of each post 16 may have incremental steps 18, as shown in Figure 1, which help to provide anchoring points for suturing, for example, or the posts may not include such steps, as shown with post 132 in Figure 3, which will be discussed in further detail below. If steps 18 are provided, they can be generally perpendicular to the vertical posts, which will make the openings generally rectangular in shape, or the steps can be differently oriented and shaped so that the openings are circular, elliptical, or another chosen shape. It is further noted that the vertical portions of the posts 16 can be made of a different material or have a different thickness than the rest of the stent wires and/or the posts can be made with reinforced attachment stents or welds on the outflow end to provide additional strength in this area.

With this stent 10, wire structure extends between one end of the post 16 and the first end 12 (which may be referred to as the aortic aspect of the stent) and additional wire structure extends between the other end of the stent post and the second end 14 (which may be referred to as the ventricular aspect of the stent). The stent 10 may include one longitudinal post 16 for each commissure of the valve that will be attached thereto, if desired. That is, for a three-leaflet valve, three longitudinal posts 16 will be provided.

The stent 20 of Figure 2 includes multiple wires that are arranged in a generally similar configuration to that discussed above relative to Figure 1. However, stent 20 further includes a central bulbous region between its first and second ends 22, 24 that is larger in diameter than the diameters of the first and second ends of the stent. The bulbous region can be configured to generally match the contours of the anatomy where the stent will be positioned in the patient (e.g., at the aortic valve sinus region). The first end 22 is flared inwardly (i.e., toward the central axis of the stent), preferably by an amount that is enough to be atraumatic, but not so pronounced that it loses contact with the patient's anatomy or interferes with another device (e.g., a coronary catheter) at a later date. Thus, the inward flare can be less than that shown, although it is possible that the flare is even greater than that shown. In addition, the second end 24 is slightly flared outwardly, as shown in the Figure. This flare at the second end 24 of the stent 20 (i.e., away from the central longitudinal axis of the stent) can prevent or minimize leakage between the implanted heart valve and the native annulus and/or to provide a physical and/or visual docking feature to secure the stent against a wall of a vessel or opening in the heart to prevent migration of the stent, for example. Additionally, the second end 24 can also have an at least slightly inward bend (see Figure 3, for example) that may be advantageous when implanting this stent in the aortic region in order to minimize trauma to adjacent anatomical structures (e.g., the mitral valve anterior leaflet or the left ventricular wall). This slight inward bend can also help to minimize pressure on the septum in the area of the bundle branch, which can in turn reduce the potential for arrythmias or heart block during or after the transcatheter valve replacement procedure.

Figures 3 and 4 illustrate stents 30 and 40 that are "selectively" flared to match particular desired shapes for portions of the stent. For example, certain stent wires are flared outwardly to avoid potential interference between the stent and the tissue leaflets of the replacement valves. The stent features to which the tissue will be attached may not be flared at all, such that the stent is relatively tubular, or these wires could instead be flared inwardly or outwardly. Stents 30, 40 include central regions 34, 44, respectively that are somewhat larger in diameter than the adjacent portions of the stent. The stent 30 further includes at least one longitudinal element or feature that can be used for attachment of tissue to the stent, such as a longitudinal post 32. Such posts 32 can also be positioned at the same distance from the longitudinal axis as the other stent elements in the central region 34, or the longitudinal posts can be closer to or further from the central axis of the stent than the other stent elements in the central region 34, if desired. By positioning the posts closer to the central axis than the other wires in the central region 34, the free edges of the dynamic leaflets positioned inside the stent 30, 40 of the new or replacement valve would be less likely to contact the stent posts when the valve leaflets are fully open. This reduced contact can reduce the potential for wear on the leaflets during valve cycling. An additional benefit of positioning the wires of the post closer to the central longitudinal axis as the other stent wires is to minimize stress at the commissures, and to help maintain coronary perfusion. This can be accomplished by limiting the opening of the leaflet so that coronary flow behind the valve leaflets is maintained. Yet another benefit of these configurations having attachment points that are inwardly offset from the largest outer diameter of the stent is that a smaller tissue valve can be used, which in turn reduces the overall transcatheter crimp profile of the delivery system.

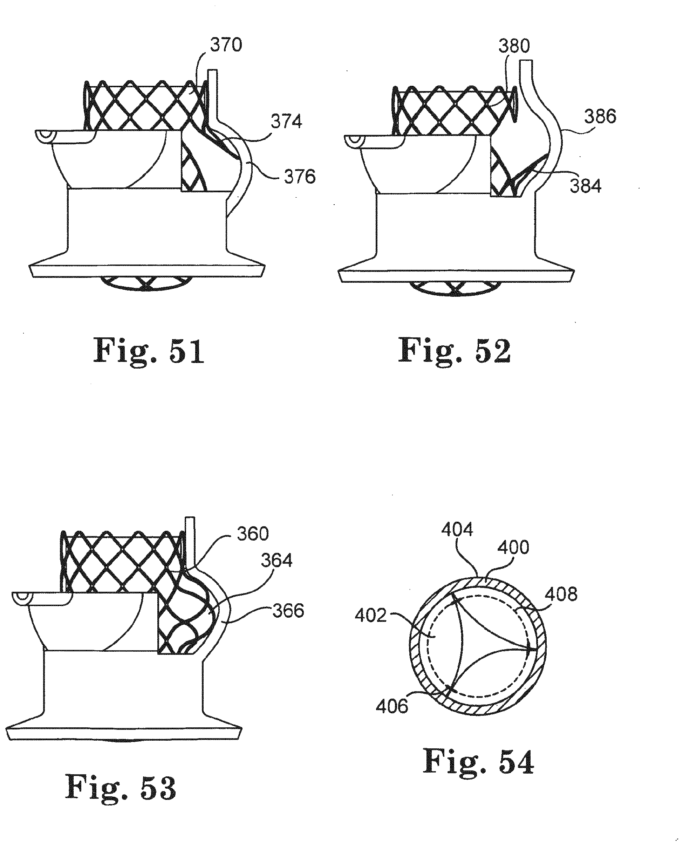

Reduction of the potential wear on the valve leaflets can alternatively be accomplished by fastening leaflet commissures closer to the center of the stent than to the outer circumference. Figure 54 illustrates such an arrangement with a schematic view of an outer stent frame 400 having three leaflets 402 arranged so that each two adjacent leaflets are attached to the stent frame 400 at a leaflet commissure area 404. Another fastening point of each of these sets of leaflets 402 at the commissure area 404 is shifted inwardly toward the center of the stent frame 400 to an inner fastening point 406. In this way, when the leaflets 402 are open, their free edges can only move out as far as the circle or inner area 408, which is shown schematically with a broken line. As shown, even if the leaflets 402 are in this fully open position, they will not contact the outer stent frame 400, thereby reducing potential wear on the valve leaflets.

Stents 10, 20, and 40 each include an arrangement of wires that provides twelve stent crowns at one end and six stent crowns at the opposite end, while stent 30 includes twelve crowns at both ends. For embodiments that include twelve crowns at the inflow end of the stent, this configuration can provide additional strength to the stent annulus area to prevent migration, to open stenotic native valve orifices, and also to provide a greater number of points for attaching pericardial leaflets to the stent. It is possible, however, to provide less than twelve (e.g., six) crowns at the outflow because the same stent strength is not required at this end for less tissue attachment points are needed. These illustrated stents are only some of the arrangements of wires that can achieve this feature of having different numbers of stent crowns at opposite ends of a single stent. In a further alternative, each of the ends of one stent can have the same number of stent crowns, but the center portion can have a more or less dense concentration of wires than either of the ends. In any case, a stent having less stent crowns at one of its ends may simplify the use of an associated delivery system, since the end with less stent crowns will have a corresponding smaller number of crowns that need to be connected to the delivery system.

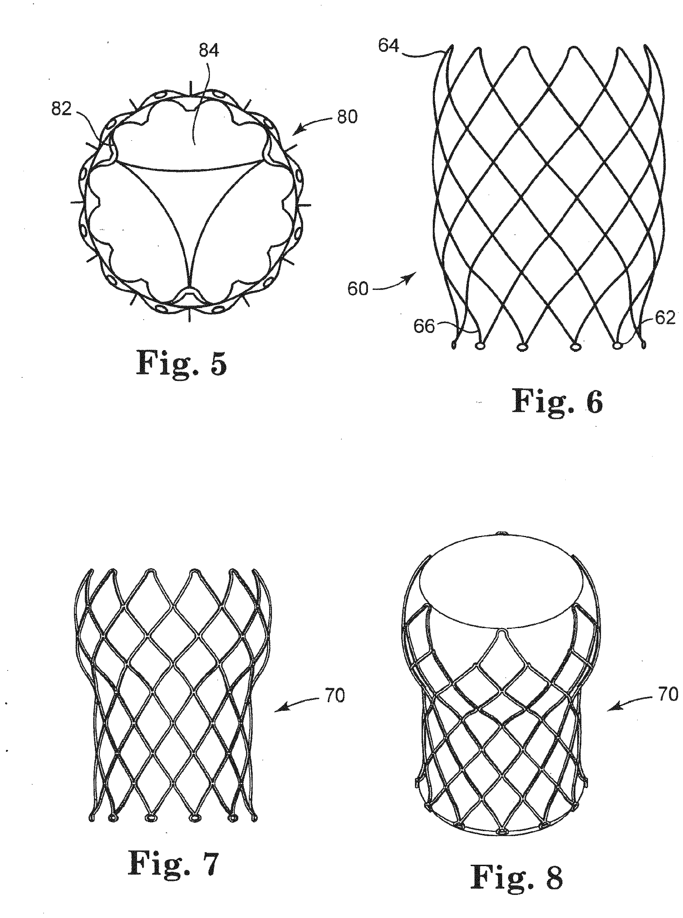

Figures 5-8 illustrate additional stent embodiments 80, 60, 70. Stent 60 has a similar shape to the stent 20 of Figure 2; however, stent 60 includes the same number of stent crowns at both ends, and also does not have the same longitudinal posts that are part of the wire arrangement of stent 20. Rather, stent 60 includes a generally regular diagonal criss-cross wire pattern along its entire length, and further includes multiple eyelets or hooks 62 at one end. A first stent end 64 is flared generally inwardly and a second stent end 66 is contoured both inwardly and outwardly as compared to the central region of the stent. Stent 70 includes a bulbous shape to the wires at one end, eyelets or hoops at the opposite end, and differing numbers of stent crowns at the opposite ends of the stent. Stent 80 includes pocket portions 82 that provide attachment points for the leaflets 84 that are positioned inwardly from the outer diameter of the stent 80. Again, these inwardly located attachment points will reduce the potential for leaflet abrasion and moves the commissure attachment points to an area that that puts less stress on the leaflets. Finally, the pockets 82 provide an area where the suture knots can be positioned so that they do not increase the overall crimp profile of the valve.

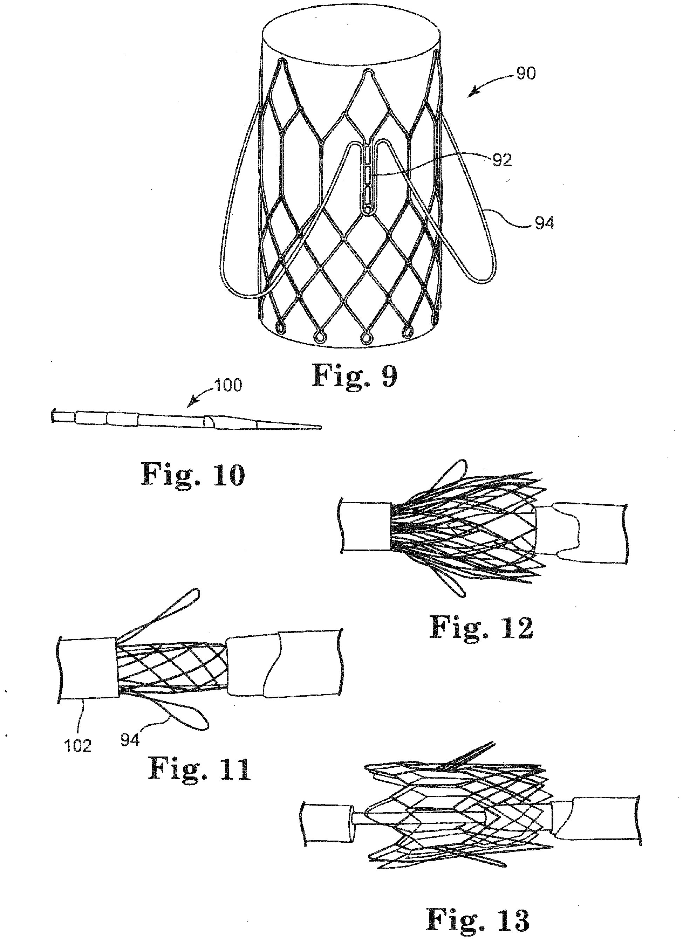

Figure 9 illustrates another stent embodiment 90 that includes several features described above relative to stent crowns, longitudinal posts, incremental steps on at least one of the posts and the wire. Stent 90 also includes at least one longitudinal stent post 92 comprised of two vertical bars spaced from each other. Stent 90 further includes three wings 94, each of which extends outwardly from the stent body and between two longitudinal posts 92. The longitudinal posts 92 can be positioned inwardly of the outer diameter of the stent 90 to provide the advantages discussed above relative to avoiding leaflet abrasion and the like. These wings can be used to dock the stent against the top aspect of the native leaflets when the stent is implanted. Again, this stent has differing numbers of crowns at its opposite ends, and hooks or eyelets on the crowns at one end.

Figures 10-13 illustrate a portion of one exemplary delivery system 100 for delivering a stent having wings, such as stent 90. In particular, Figure 10 shows a delivery system tip including a fully crimped stent enclosed within a main catheter sheath. Figure 11 shows the wings 94 being deployed from the delivery system 100 by retracting the main catheter sheath 102. In an implantation, the wings 94 can be positioned to interface with the outflow aspect of the native valve leaflets. Once these wings 94 are in contact with the native valve leaflets, the inflow or annular end of the stent is deployed by driving the catheter tip forward, as illustrated in Figure 12. The native leaflets will now contact the wings 94 and inflow end of the stent 90, thereby minimizing the potential for migration of the replacement valve. The outflow end of the stent can now be deployed, as shown in Figure 13, to fully re-expand the stent 90 release it from the delivery system, which is accomplished by further retracting the main catheter sheath.

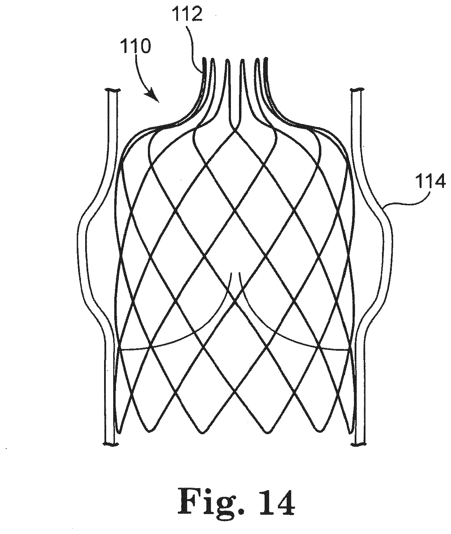

Figure 14 illustrates a stent 110 that includes a highly flexible delivery system attachment end 112 that enables the portion of the stent 110 that interfaces with the anatomy to create secure fixation to be fully deployed while still attached to the delivery system. This system enables a sprocket-style delivery system attachment mechanism that can help to minimize the delivery system diameter size. A sprocket-style delivery system includes some type of inner core member from which multiple protrusions extend, where the shape of the protrusions allow for engagement with wires of a stent. Stent 110 does not require attachment of each crown on the aortic end of the stent, while still enabling the ventricular region of the stent to fully deploy to assess functionality and positioning, which can thereby allow for a smaller diameter for the delivery system. As shown, stent 110 is positioned relative to an aorta 114, and stent 110 includes an outflow end that has very flexible struts that enable the anchoring portion of the stent to be fully deployed to assess the valve functionality and positioning, while still being captured on a sprocket-style delivery system. The outer diameter of the stent can preferably expand to match the maximum inner diameter of the anchoring region.

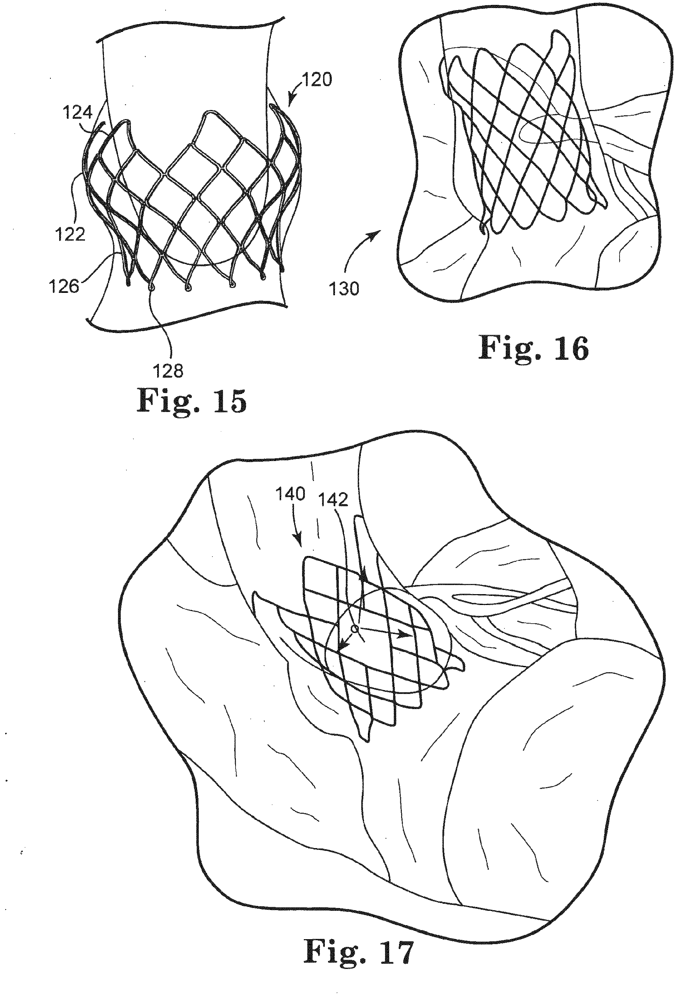

Figure 15 illustrates another embodiment of a stent 120 having a central region 122 with a diameter that is larger than the diameter at either of the ends. A first end 124 has six stent crowns, while the opposite second end 126 has twelve stent crowns, each of which includes an eyelet 128. With such an arrangement, the number of crowns provided at the outflow end of the stent is reduced, thereby requiring fewer points for attachment to a delivery system. Figure 16 illustrates another stent embodiment 130 including flared regions at both ends and a central region that is generally cylindrical.

Figure 17 illustrates another embodiment of a stent 140 that is positioned at the aortic valve position of a heart. Stent 140 includes six stent crowns at one end and twelve stent crowns at the opposite end, and further includes a central area with a relatively large opening or gap 142 between the wires. The gap 142 can be positioned at the coronary ostia so as to not obstruct or interfere with blood flow. The stents 120, 130, 140, along with many of the other stent embodiments described herein, are designed to match with native anatomic features of a patient to improve resistance to migration and improve paravalvular replacement valve sealing.

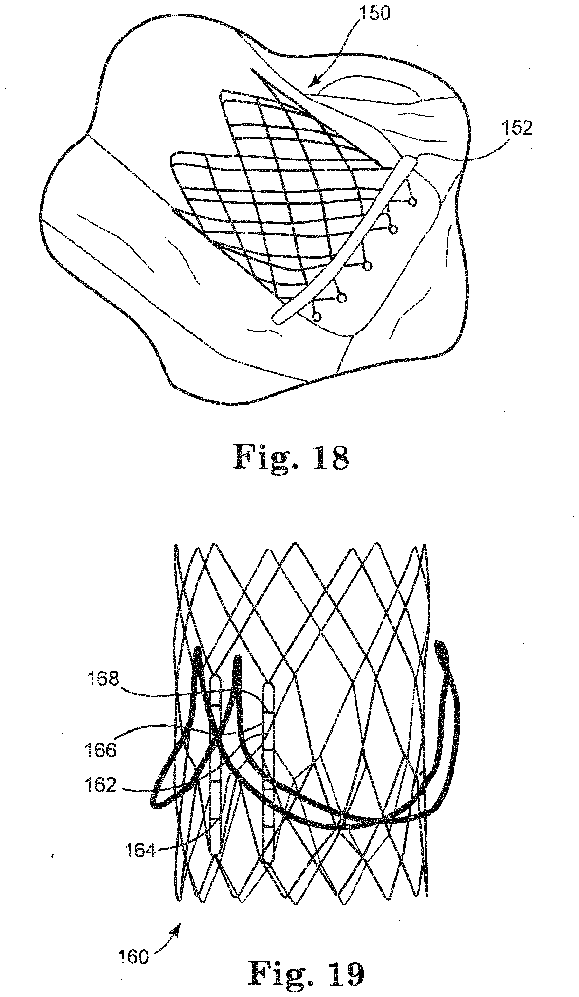

Figure 18 illustrates another embodiment of a stent 150 that is designed for anatomic compatibility and includes a bulbous portion that is positioned to sit generally at the annular area of a vessel. A ring 152 shown in this figure is a sealing gasket on the outside of the stent and is positioned generally at the annulus of a vessel when implanted. The gasket can be made of fabric or inflatable tube structures, for example.

Figure 19 illustrates a stent 160 that does not include many of the contours described relative to other stent embodiments of the invention, but includes longitudinal posts 162 for attachment of the valve tissue. Posts 162 are comprised of two longitudinal wire portions 166 spaced from each other, and further include optional intermediate members 168 that extend between the longitudinal portions 166. The outer structure ring structure shown in this drawing is provided as an illustration of the general stitching path that can be used for tissue material within the stent.

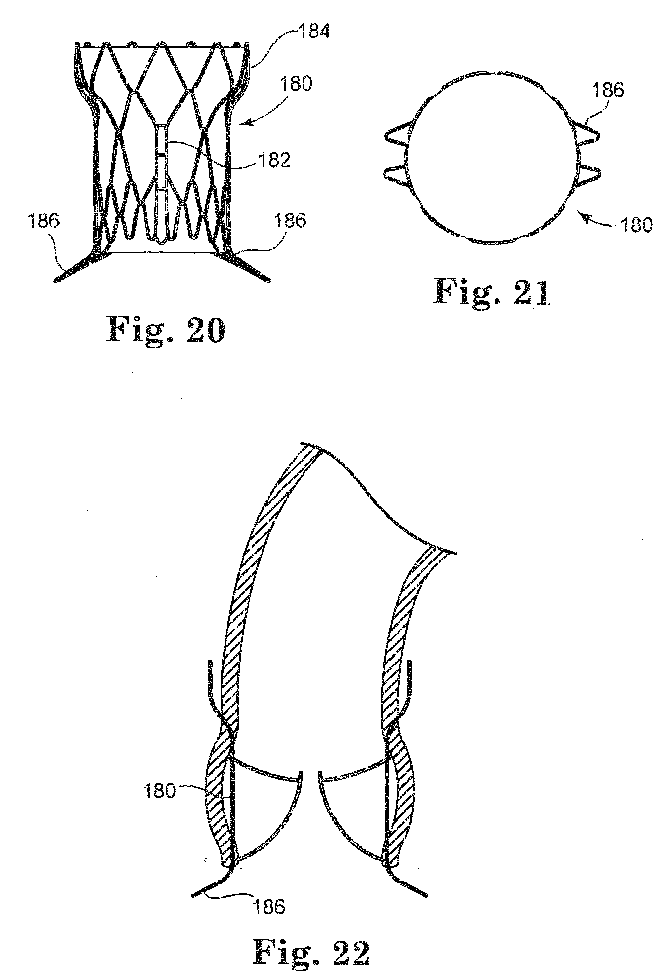

Figures 20-22 illustrate an embodiment of a stent 180 that includes a number of features described above for the stents of the invention, along with additional features. In particular, Figure 20 shows the stent 180 with longitudinal posts 182 extending in the direction of the length of the stent 180, and a region 184 at one end that is bulbous or has a larger diameter than the central portion of the stent. The opposite end of the stent 180 includes flared portions 186 that extend from opposite sides of the generally tubular central portion . As shown in Figure 21, each flared portion 186 can include two crowns, although it is possible that the flared portion 186 can be configured somewhat differently than shown (e.g., there can be more or less crowns, the crowns can be shaped differently, the flared portion 186 can extend around a larger or smaller portion of the circumference of the stent, and the like). As is further illustrated in Figure 20, the geometry of the stent can be designed to incorporate optimal attachment points for tissue. That is, the stent node trajectory can be specifically selected to provide the desired points for the attachment of tissue. Such a feature can be considered and designed for tents including longitudinal posts, as shown in Figure 20, and may also be considered for stents comprising more diamond-shaped wire patterns without longitudinal posts.

The outer profile of stent 180 is shown in an exemplary position within the anatomy (i.e., aorta) of a patient in Figure 22, with the central area that includes the commissural posts being positioned in the bulbous area of an aorta. The flares 186 extend into the ventricle in order to help anchor the stent 180 in place. The flares 186 are preferably positioned in locations where they do not disrupt the native anatomical function. That is, the flares 186 should not interfere with the mitral valve anterior leaflet and should not apply pressure to the septum in the area of the conduction system bundle branch. Again, it is also preferable that the central portion of the stent 180 does not contact the native aortic sinus region, in order to minimize the potential for coronary occlusion or obstruction.

It is noted that in many of the stent embodiments shown and described herein, the aspect ratio of certain portions of the stent is exemplary, and can be somewhat different from that shown. It is further noted that if the stent of any of the embodiments is to be positioned to replace the aortic valve, the stent can be provided with a lower density wire portion in the area where the coronaries are located. To eliminate the need to clock the device, reduced wire density around the entire perimeter of the stent in the central area can be provided. Further, stent embodiments described herein may be modified to include additional structure for attachment of tissue for the valve, such as the vertical stent posts described in many of the embodiments.

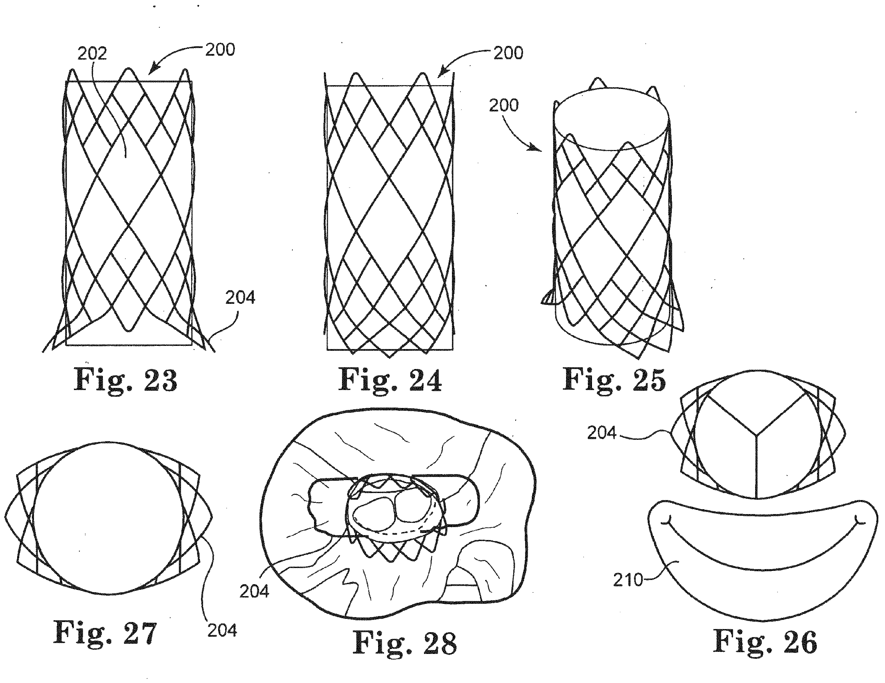

Figures 23-28 illustrate another embodiment of a stent 200 that includes a central cylindrical portion with at least two regions with a lower density of wires, each of which is provided for positioning in the area of the coronary openings. The wires of this lower density area are arranged to provide openings 202 that are larger than the spaces between other wires of the stent. These openings are offset along the length of the stent to be arranged in a zigzag type of pattern around the circumference of the stent 120. One end of the stent 200 includes flared portions 204 that extend from opposite sides of the central cylindrical portion of the stent. Each flared portion 204 includes three crowns, although variations of this configuration are contemplated, as discussed above relative to flared stent portions. As shown in Figure 26, stent 200 is positioned relative to a mitral valve 210 so that one of the flared portions is positioned at the left ventricle, and one of the openings in the stent is positioned at the left coronary artery. Figure 27 is a top view of the stent 190, and Figure 28 shows one exemplary position of the stent 190 relative to the anatomy of a patient, including the septum and anterior leaflet of the mitral valve.

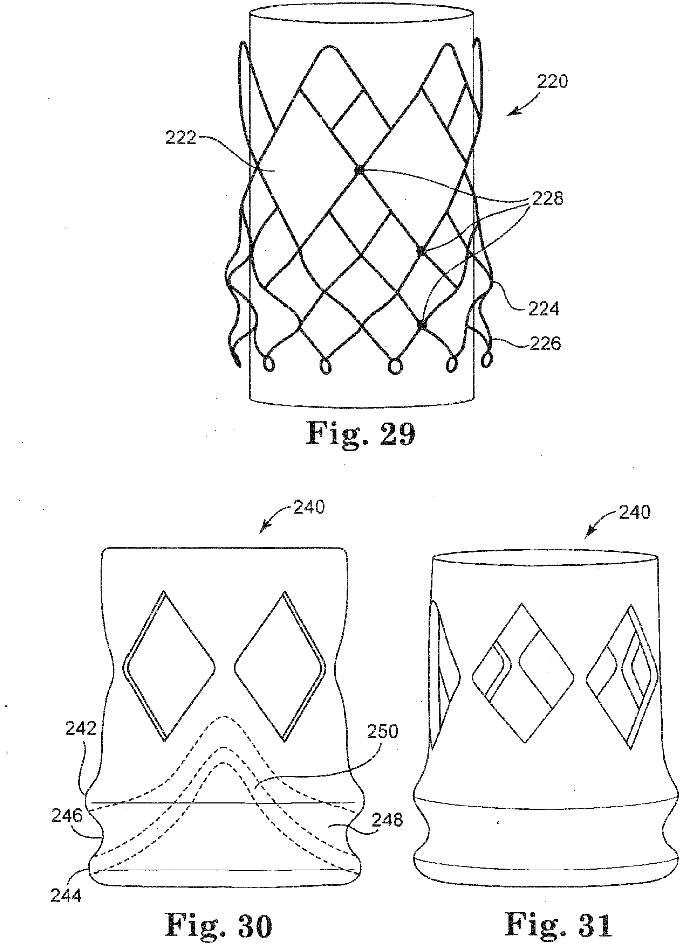

Figure 29 illustrates a stent 220 that includes openings 222 (i.e., areas of lower wire density) for the coronaries, and further includes sub-annular and supra-annular circumferential wings to help secure the stent to the patient's native anatomy. In particular, the area below the openings 222 includes an outward curve or flare to create a wing 224 that can extend around all or part of the circumference of the stent 220. The wires then curve back toward the central longitudinal axis of the stent, then curve or flare outwardly again to create a wing 226 that can extend around all or a part of the circumference of the stent 220. As shown, the wings 224, 226 and the area between them form a generally sinusoidal configuration, where the wing 224 can be positioned above an annulus and wing 226 can be positioned below that annulus to provide the anchoring for a more secure attachment in that position. This series of wings can help to anchor the stent in regions of calcified or fused leaflets in the aortic stenosis patient population. Stent 220 further includes imaging markers 228 that can be used to identify the high and low points of the commissures, the annular (valve) plane of the implant, and/or other features. Markers can also be used to identify high and low boundaries for optimal implant placement within the patient's anatomy.

Figures 30 and 31 are solid models of a stent 240 that is configured similarly to the stent of Figure 29, including the sinusoidal shape at one end that creates wing areas. These wings can have a different profile from that shown, although it is preferable in this embodiment that there are sinusoidal "peaks" 242, 244 that are separated by a "valley" 246, where the annulus of a valve can be positioned in the valley 246 so that the peaks 242, 244 are on opposite sides of the annulus. The peaks and valleys can have different heights than shown, and the spacing between the peaks may also be different. That is, the spacing between the sub-annular and supra-annular flares can be varied, depending on the specific procedure that will be performed and the desired characteristics of the stent. These embodiments, along with other shaped stents described herein, can help to minimize stent migration within the patient due to the ability of the stent to conform to various contours of the patient's anatomy. Figure 30 also illustrates an optional groove 248 that can be positioned generally around the periphery of the stent 240 to match the native 3-dimensional configuration of the native anatomy. A gasket 250 can be positioned within the groove 248, where such a gasket 250 can include one continuous structure that generally follows the shape of the groove 248, or it can include one or more pieces within portions of the groove 248. The gasket 250 can improve paravalvular sealing. Further, the gasket 250 can be made of a material that can heal into the native tissue of the patient, which can help the stent to resist migration.

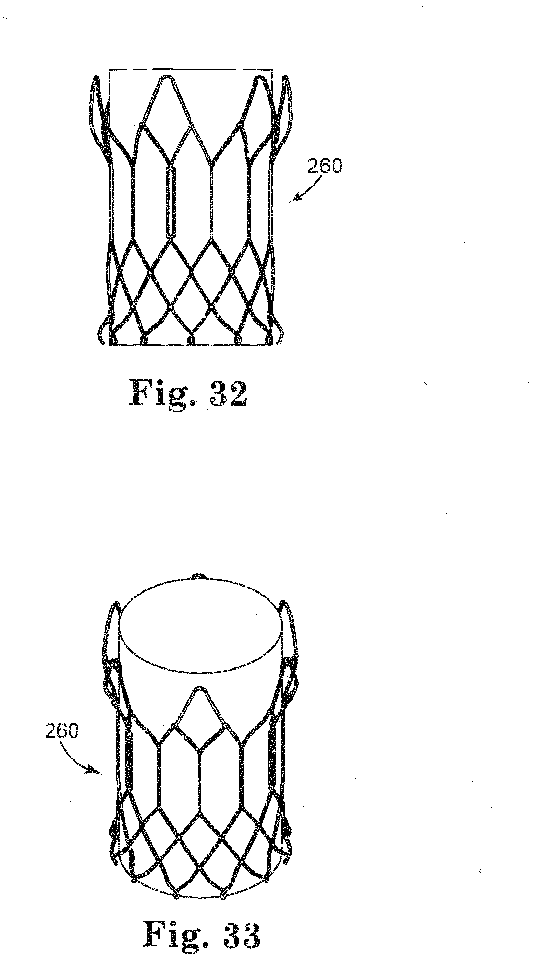

Figure 32 and 33 illustrate another stent embodiment 240 that includes flares at both the sub-annular and sinotubular junction (STJ) areas. The illustrated stent further includes vertical stent posts, twelve inflow crowns and six outflow crowns although there could be more or less than these numbers of inflow and outflow crowns. Stent 240 has a wire arrangement similar to that shown for the stents of Figures 1-4 and other stents described and shown herein; however, the central area of stent 240 is more tubular or "straight," with slightly curved areas at both ends.

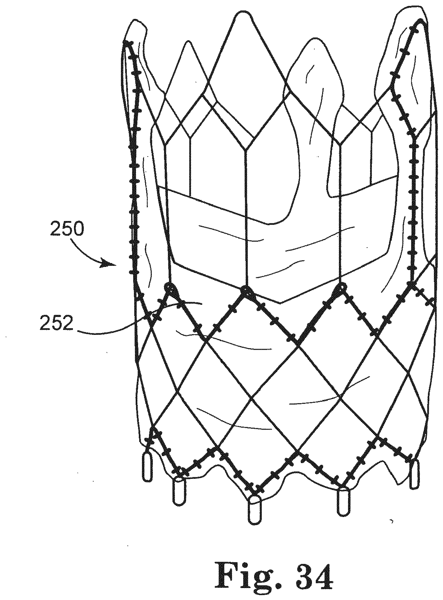

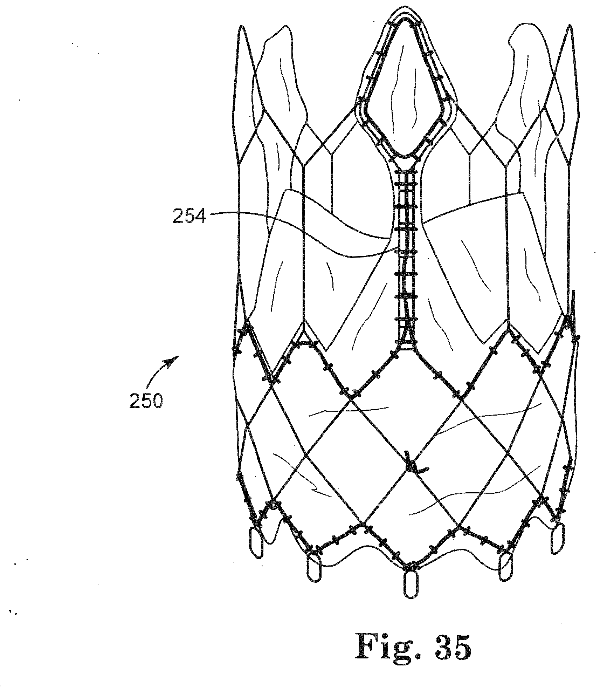

One exemplary stent of the invention combines the following features: eyelets at one end for attachment to the delivery system and tissue valve; vertical commissural tissue attach struts or posts; moderately flared non-commissural attach vertical struts or STJ flare; sub-annular flares; inflow and outflow atraumatic curvatures; a twelve crown inflow; and six tapered crowns at the outflow end. Such an embodiment of a stent is illustrated, for example, as stent 250 in Figures 34 and 35. Stent 250 further includes tissue material 252 attached within its internal area to provide leaflets for the valve. Two spaced-apart vertical members are used to make up vertical posts 254, one of which is most visible in Figure 35. One exemplary pattern for stitching the tissue to the vertical post 254 is also illustrated, although the stitching pattern can differ from that shown.

Delivering any balloon-expandable stents of the invention to the implantation location can be performed percutaneously. In general terms, this includes providing a transcatheter assembly, including a delivery catheter, a balloon catheter, and a guide wire. Some delivery catheters of this type are known in the art, and define a lumen within which the balloon catheter is received. The balloon catheter, in turn, defines a lumen within which the guide wire is slideably disposed. Further, the balloon catheter includes a balloon that is fluidly connected to an inflation source. It is noted that if the stent being implanted is the self-expanding type of stent, the balloon would not be needed and a sheath or other restraining means would be used for maintaining the stent in its compressed state until deployment of the stent, as described herein. In any case, for a balloon-expandable stent, the transcatheter assembly is appropriately sized for a desired percutaneous approach to the implantation location. For example, the transcatheter assembly can be sized for delivery to the heart valve via an opening at a carotid artery, a jugular vein, a sub-clavian vein, femoral artery or vein, or the like. Essentially, any percutaneous intercostals penetration can be made to facilitate use of the transcatheter assembly.

Prior to delivery, the stent is mounted over the balloon in a contracted state to be as small as possible without causing permanent deformation of the stent structure. As compared to the expanded state, the support structure is compressed onto itself and the balloon, thus defining a decreased inner diameter as compared to an inner diameter in the expanded state. While this description is related to the delivery of a balloon-expandable stent, the same basic procedures can also be applicable to a self-expanding stent, where the delivery system would not include a balloon, but would preferably include a sheath or some other type of configuration for maintaining the stent in a compressed condition until its deployment.

With the stent mounted to the balloon, the transcatheter assembly is delivered through a percutaneous opening (not shown) in the patient via the delivery catheter. The implantation location is located by inserting the guide wire into the patient, which guide wire extends from a distal end of the delivery catheter, with the balloon catheter otherwise retracted within the delivery catheter. The balloon catheter is then advanced distally from the delivery catheter along the guide wire, with the balloon and stent positioned relative to the implantation location. In an alternative embodiment, the stent is delivered to an implantation location via a minimally invasive surgical incision (i.e., non-percutaneously). In another alternative embodiment, the stent is delivered via open heart/chest surgery. In one embodiment of the stents of the invention, the stent includes a radiopaque, echogenic, or MRI visible material to facilitate visual confirmation of proper placement of the stent. Alternatively, other known surgical visual aids can be incorporated into the stent. The techniques described relative to placement of the stent within the heart can be used both to monitor and correct the placement of the stent in a longitudinal direction relative to the length of the anatomical structure in which it is positioned.

Once the stent is properly positioned, the balloon catheter is operated to inflate the balloon, thus transitioning the stent to an expanded state. Alternatively, where the support structure is formed of a shape memory material, the stent can self-expand to its expanded state.

One or more markers on the valve, along with a corresponding imaging system (e.g., echo, MRI, etc.) can be used with the various repositionable delivery systems described herein in order to verify the proper placement of the valve prior to releasing it from the delivery system. A number of factors can be considered, alone or in combination, to verify that the valve is properly placed in an implantation site, where some exemplary factors are as follows: (1) lack of paravalvular leakage around the replacement valve, which can be advantageously examined while blood is flowing through the valve since these delivery systems allow for flow through and around the valve; (2) optimal rotational orientation of the replacement valve relative to the coronary arteries; (3) the presence of coronary flow with the replacement valve in place; (4) correct longitudinal alignment of the replacement valve annulus with respect to the native patient anatomy; (5) verification that the position of the sinus region of the replacement valve does not interfere with native coronary flow; (6) verification that the sealing skirt is aligned with anatomical features to minimize paravalvular leakage; (7) verification that the replacement valve does not induce arrhythmias prior to final release; and (8) verification that the replacement valve does not interfere with function of an adjacent valve, such as the mitral valve.

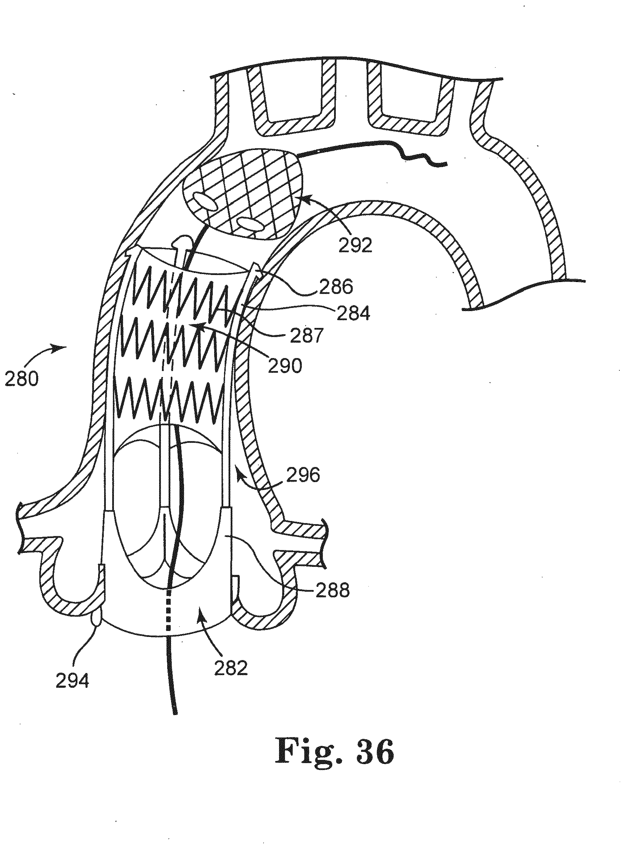

Figures 36-39 are schematic views of various embodiments of stents of the present invention. In particular, Figure 36 illustrates a stent assembly 280 that includes features that align and secure it with specific anatomical features in the left ventricle region and the left ventricular outflow tract region of a patient. Stent assembly 280 includes a stented valve 282 from which tethers 284 extend. Tethers 284 are preferably flexible to accommodate curvature of the native aorta above the valve annulus. Optional anchors 286 are shown at the distal ends of the stent. More specifically, each of the tethers 284 extends from one of the commissures 288 of the stent 282. The stent assembly 280 further includes a distal element such as a stent graft 290 positioned between the tethers 284 near the anchors 286, which is flexible and can accommodate widely varying patient anatomy. The stent graft 290 will be positioned distal to the sinus area of the left ventricular outflow tract when implanted. This configuration can facilitate stabilization of the stent assembly and may be designed to register or interface with another stent graft that is implanted at a later time.

This stent assembly 280 can include flexible connections between annular and supra-annular stent aspects. The flexible connections may be elastomeric, fabric, metal, or the like. Such flexible connections can help the stent assembly to accommodate most varying anatomy above the sinotubular junction and also to accommodate aortic curvature. In addition, the flexible connections can make the stent assembly able to accommodate anerysmal aortas.

The stent assembly 280 may further include a gasket 294 positioned adjacent an end of the stented valve 282. In addition, when the stent assembly is implanted in a patient, a plaque pocket 296 can be created that provides embolic protection by creating a volume that can entrap plaque, calcification, and other emboli from traveling in a distal direction and causing a thromembolic event, such as a stroke.

Alternatively, portions of the system may be designed to include a longer useful life than others. For example, the frame of the present invention could be designed to have a relatively long useful life (e.g. 20 years), while the tissue component could have a relatively shorter useful life (e.g. 10 years).

An embolic protection device 292 can be provided distal to the stent assembly 280, as is shown in Figure 36. The device 292 can be utilized during the implantation procedure to capture and trap any emboli released and/or generated by the valve procedure, while still maintaining uninhibited or sufficient perfusion through the aorta and coronary arteries during valve implantation. In addition, Figures 36-39 illustrate a portion of the stent positioned above the sinotubular junction 284 covered with fabric, polymer, and/or tissue, which can serve this same purpose.

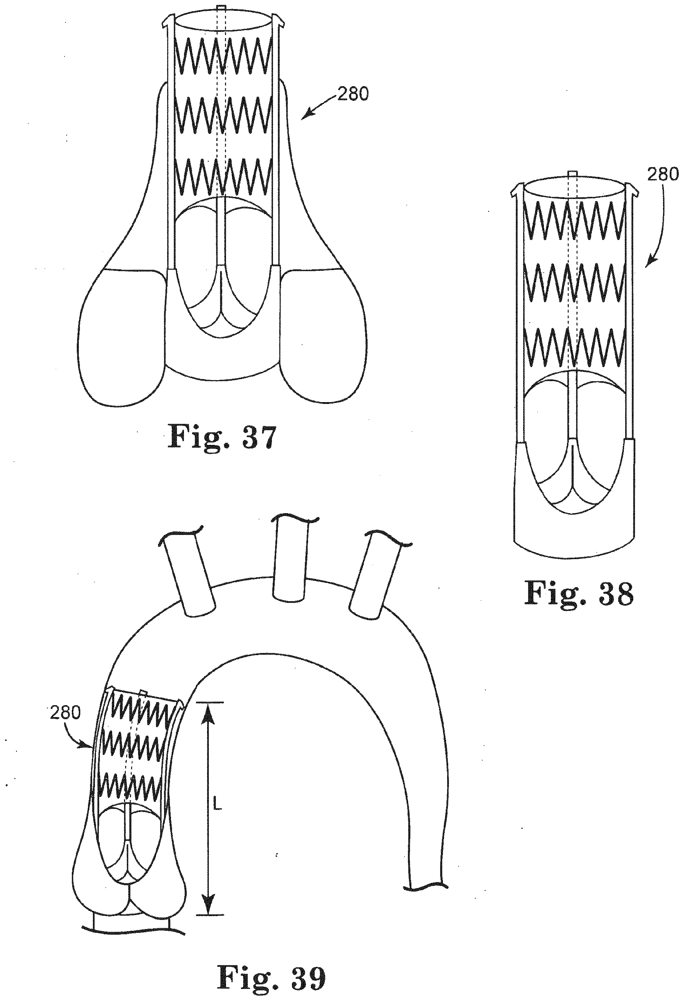

Figures 37-39 illustrate alternative views of the stent assembly 280, both within a heart vessel and independent of anatomical structure (Figure 38). It is noted that the anchoring of the stent posts via the anchors 286 can help to prevent valve ejection. Figure 37 shows the stent assembly 280 implanted in a supra-annular position in a patient's anatomy, which can beneficially improve the orifice area by avoiding the stenotic region of the aorta. Figure 39 shows the flexibility of the stent graft material in order to conform to the curved area of an aorta.

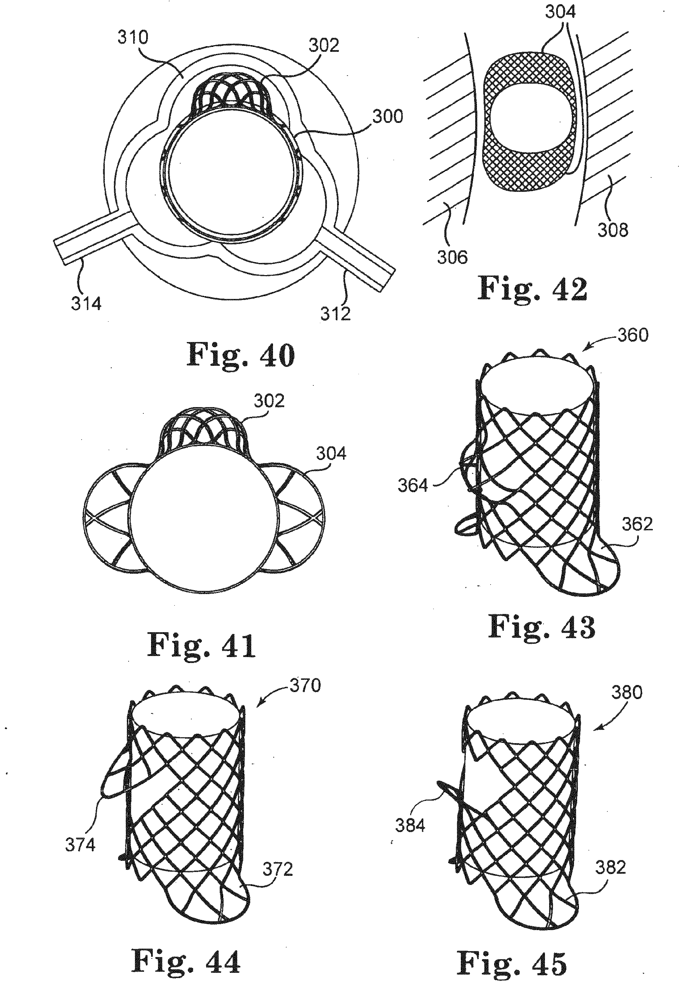

Figure 40 illustrates a top view of a stent 300 having a fixation tab 302 positioned in the non-coronary sinus area 310, and with no such tabs at either the right coronary artery 314 or the left coronary artery 312. That is, fixation components of stent 300 may secure the system to non-coronary sinus and/or regions of the left ventricle adjacent to the aortic valve annulus. This may avoid obstruction of coronary blood flow and prevent unwanted interaction between the system and the septum and mitral valve anterior leaflet. Further, the fixation tab 302 does not prevent or inhibit subsequent coronary intervention, while providing the advantage of minimizing or preventing migration of the stent toward the aorta. Figure 41 illustrates a stent having both a fixation tab 302 and flared portions 304 that help to prevent migration of the stent. Figure 42 illustrates stent 300 having flared regions 304 as positioned relative to the interventricular septum 306 and the mitral valve apparatus 308.

Figures 43-45 illustrate alternative stent embodiments 360, 370, 380, each of which comprises an extending or fixation tab 364, 374, 384, respectively, along with flared portions 362, 372, 382, respectively. Tab 364 of stent 360 is configured as a bulging wire area, tab 374 of stent 370 comprises an extension that is angled in the same general direction as the wings 372, and tab 384 of stent 380 comprises an extension that is angle in generally the opposite direction from that of the wings 382. The stent 370 is illustrated in Figure 51 with its tab 374 positioned relative to a non-coronary sinus 376, stent 380 is illustrated in Figure 52 with its tab 384 positioned relative to a non-coronary sinus 386, and stent 360 is illustrated in Figure 53 with its fixation tab 364 positioned relative to a non-coronary sinus 366. As shown, these tabs can help to prevent stent migration due to their interference with the patient's anatomy.

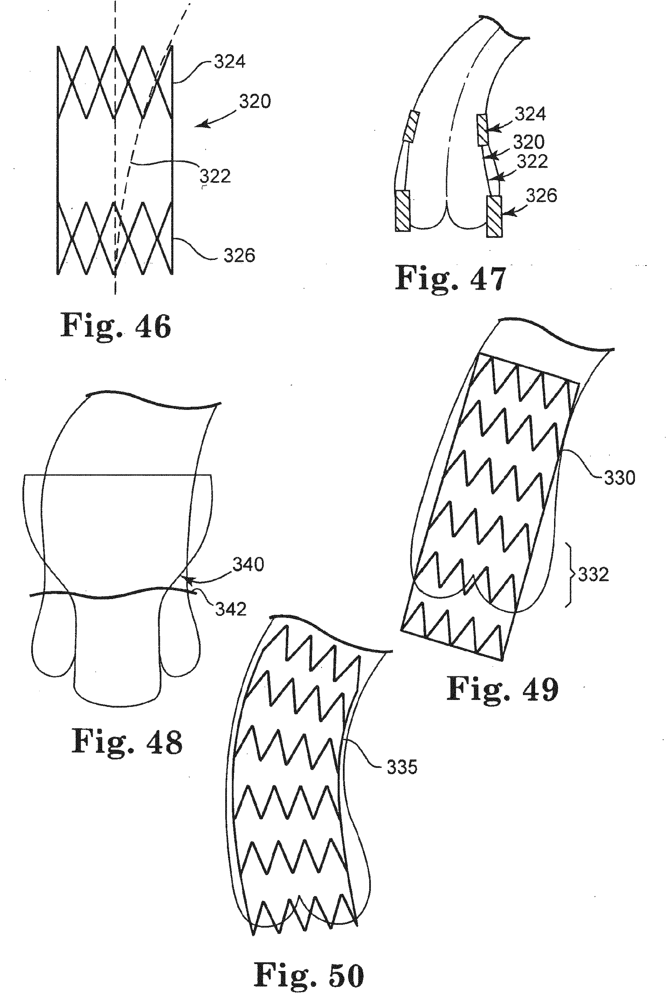

Figures 47 and 48 schematically illustrate the aorta of a patient. As shown, the aorta begins to curve distal to the annulus level. Many typical transcatheter valve stents are cylindrical with a relatively straight axis. Such a stent structure does not easily conform to the native anatomy, which can present a number of potential issues. First, the reduced pressure on the anatomy at the inner portion of the curvature (such as is illustrated with the an area 332 adjacent to a stent 330 in Figure 49) can lead to improper seating, migration, and/or paravalvular leakage. Second, increased pressure on the anatomy at the outer portion of the curvature can lead to, or increase the potential for cardiac conduction system block or interference. Third, increased pressure on the anatomy at the outer portion of the curvature can lead to local erosion, irritation, and/or dissection of tissue. Fourth, the stent can be subjected to increased torsional and/or bending stresses and strains, which can affect the short-term structural integrity of the stent. Finally, lack of conformity with the curvature of the native anatomy can inhibit the ability of the clinician to accurately or consistently position the stent/valve in the desired location.

Several stents of the present invention can alleviate this nonconformity of the valve frame with the native anatomy. In one embodiment, the stent could have a predetermined curvature that matches or more closely conforms to the native anatomy, such as stent 335 in Figure 50. In other embodiments, the stent could have flexibility (e.g., area 322 of stent 320 in Figures 47-48) or a hinged area (e.g., hinge 342 of stent 340 in Figure 48) in the portion of the stent that would enable it to conform to the native curved anatomy. Figures 46 and 47 illustrate stent designs that incorporate flexibility in their central regions, which in turn enables improved conformity with the native anatomy. The central areas or members 322 can be fabricated from a wide variety of materials, such as metals, polymers, fabrics, and the like. The members 322 can include a number of geometries that allow flexibility to conform to the native, curved aortic anatomy. Referring again to Figure 36, this stent assembly incorporates elements 287 that are not attached to each other except through flexible materials such as fabric, tissue, or polymeric materials that enable a high degree of conformity with the native anatomy curvature within the ascending aorta.

The present invention also optionally or alternatively includes distal emoboli protection features which may be incorporated into a delivery system for delivering a stent assembly (e.g. in the nose assembly), such as the thromboembolic filter. The protection features may provide acute protection during percutaneous valve delivery. The protection features may afford substantially uninhibited flow through coronaries during systole or diastole.

## Claims

1. A stented valve comprising:
a stent structure (10, 20, 30, 40) comprising a generally tubular body portion having a first end (12, 22) and a second end (14, 24),
and a valve structure attached within the generally tubular body portion, wherein the valve structure comprises leaflets,
wherein the first end (14, 24) of the stent structure (10, 20, 30, 40) is an inflow end and the second end (12, 22) of the stent structure (10, 20, 30, 40) is an outflow end, and wherein the stent structure (10, 20, 30, 40) comprises a number of strut or wire portions, wherein the arrangement of wires is such that the stent structure comprises twelve crowns at the inflow end of the stent structure (10, 20, 30, 40) and less crowns at the outflow end of the stent structure (10, 20, 30, 40),
wherein the stent structure (10, 20, 30, 40) further includes three longitudinal posts (16, 32) for attachment of the valve structure, wherein the three longitudinal posts (16, 32) for attachment of the valve structure are comprised of two bars or vertical portions that are spaced from each other by a sufficient distance to allow leaflets to be drawn between the vertical portions at the leaflet commissures, and
wherein the stent structure (10, 20, 30, 40) includes a wire structure that extends between one end of each post (16, 32) and the first end (14, 24) of the stent structure (10, 20, 30, 40) and an additional wire structure that extends between the other end of each post (16, 32) and the second end (12, 22) of the stent structure (10, 20, 30, 40).

2. The stented valve according to any of the preceding claims, wherein the posts (16, 32) for attachment are closer to a central axis of the stent structure than other stent elements.

3. The stented valve according to any of the preceding claims, wherein the stent structure includes a central region (34) that is larger in diameter than adjacent regions of the stent structure (10, 20, 30, 40), and wherein the posts (16, 32) for attachment are closer to a central axis of the stent structure than other stent elements in the central region (34) of the stent structure (10, 20, 30, 40).

4. The stented valve according to any of the preceding claims, wherein a center portion of the stent structure (10, 20, 30, 40) is provided with a lower density wire portion.

5. The stented valve according to claim 4, wherein the stented valve is configured to be positioned to replace an aortic valve, and wherein the lower density wire portion is provided in the area of the stent structure (10, 20, 30, 40) positioned where the coronary arteries are located when the stent structure (10, 20, 30, 40) is implanted.

6. The stented valve according to any of claims 4 or 5, wherein the lower density wire portion extends around the entire perimeter of the stent structure (10, 20, 30, 40).

7. The stented valve according to any of claims 4 to 6, wherein the wires of the lower density wire portion are arranged to provide openings that are larger than the spaces between other wires of the stent structure (10, 20, 30, 40).

8. The stented valve according to any of the preceding claims, wherein the stent structure (10, 20, 30, 40) is compressible for percutaneous delivery and expandable by removal of external compressive forces.

9. The stented valve according to any of the preceding claims, wherein the stent structure (10, 20, 30, 40) is a self-expanding stent.

10. The stented valve according to any of the preceding claims, wherein the stent structure (10, 20, 30, 40) includes a radiopaque material, echogenic material or **MRI** visible material.

11. The stented valve according to any of the preceding claims, wherein the wires of the stent structure (10, 20, 30, 40) are formed from a shape memory material such as a nickel titanium alloy, e.g. Nitinol.

12. The stented valve according to any of the preceding claims, wherein the leaflets are pericardial leaflets.

13. A delivery system comprising:
the stented valve according to claim 8, and
a catheter with a retractable sheath that covers the stent structure (10, 20, 30, 40) until it is to be deployed, wherein the sheath can be retracted to allow the stent structure (10, 20, 30, 40) to expand.

## Patentansprüche

1. Gestentete Klappe, umfassend:
eine Stentstruktur (10, 20, 30, 40), umfassend einen im Allgemeinen rohrförmigen Körperabschnitt, der ein erstes Ende (12, 22) und ein zweites Ende (14, 24) aufweist,
und eine Klappenstruktur, die innerhalb des im Allgemeinen rohrförmigen Körperabschnitts angebracht ist, wobei die Klappenstruktur Segel umfasst,
wobei das erste Ende (14, 24) der Stentstruktur (10, 20, 30, 40) ein Einströmende und das zweite Ende (12, 22) der Stentstruktur (10, 20, 30, 40) ein Ausströmende ist, und wobei die Stentstruktur (10, 20, 30, 40) eine Anzahl von Streben- oder Drahtabschnitten umfasst, wobei die Anordnung von Drähten derart ist, dass die Stentstruktur zwölf Kronen an dem Einströmende der Stentstruktur (10, 20, 30, 40) und weniger Kronen an dem Ausströmende der Stentstruktur (10, 20, 30, 40) umfasst,
wobei die Stentstruktur (10, 20, 30, 40) ferner drei Längspfosten (16, 32) für eine Befestigung der Klappenstruktur einschließt, wobei die drei Längspfosten (16, 32) für die Befestigung der Klappenstruktur aus zwei Stangen oder vertikalen Abschnitten bestehen, die eine ausreichende Distanz voneinander beabstandet sind, um den Segeln zu ermöglichen, zwischen den vertikalen Abschnitten an den Segelkommissuren gezogen zu werden, und
wobei die Stentstruktur (10, 20, 30, 40) eine Drahtstruktur einschließt, die sich zwischen einem Ende jedes Pfostens (16, 32) und dem ersten Ende (14, 24) der Stentstruktur (10, 20, 30, 40) erstreckt, und eine zusätzliche Drahtstruktur, die sich zwischen dem anderen Ende jedes Pfostens (16, 32) und dem zweiten Ende (12, 22) der Stentstruktur (10, 20, 30, 40) erstreckt.

2. Gestentete Klappe nach einem der vorstehenden Ansprüche, wobei die Pfosten (16, 32) für die Befestigung näher an einer zentralen Achse der Stentstruktur als andere Stentelemente liegen.

3. Gestentete Klappe nach einem der vorstehenden Ansprüche, wobei die Stentstruktur einen zentralen Bereich (34) einschließt, der einen größeren Durchmesser als angrenzende Bereiche der Stentstruktur (10, 20, 30, 40) vorweist, und wobei die Pfosten (16, 32) für die Befestigung näher an einer zentralen Achse der Stentstruktur als andere Stentelemente in dem zentralen Bereich (34) der Stentstruktur (10, 20, 30, 40) liegen.

4. Gestentete Klappe nach einem der vorstehenden Ansprüche, wobei ein Mittelabschnitt der Stentstruktur (10, 20, 30, 40) mit einem Drahtabschnitt geringerer Dichte versehen ist.

5. Gestentete Klappe nach Anspruch 4, wobei die gestentete Klappe konfiguriert ist, um eine Aortenklappe zu ersetzen, und wobei der Drahtabschnitt mit geringerer Dichte in dem Bereich der Stentstruktur (10, 20, 30, 40) vorgesehen ist, der dort positioniert ist, wo sich die Koronararterien befinden, wenn die Stentstruktur (10, 20, 30, 40) implantiert wird.

6. Gestentete Klappe nach einem der Ansprüche 4 oder 5, wobei sich der Drahtabschnitt mit geringerer Dichte um den gesamten Umfang der Stentstruktur (10, 20, 30, 40) herum erstreckt.

7. Gestentete Klappe nach einem der Ansprüche 4 bis 6, wobei die Drähte des Drahtabschnitts mit geringerer Dichte angeordnet sind, um Öffnungen bereitzustellen, die größer als die Zwischenräume zwischen anderen Drähten der Stentstruktur (10, 20, 30, 40) sind.

8. Gestentete Klappe nach einem der vorstehenden Ansprüche, wobei die Stentstruktur (10, 20, 30, 40) für eine perkutane Abgabe komprimierbar und durch Entfernung externer Druckkräfte expandierbar ist.

9. Gestentete Klappe nach einem der vorstehenden Ansprüche, wobei die Stentstruktur (10, 20, 30, 40) ein selbstexpandierender Stent ist.

10. Gestentete Klappe nach einem der vorstehenden Ansprüche, wobei die Stentstruktur (10, 20, 30, 40) ein röntgendichtes Material, ein echobildendes Material oder ein in einem MRI sichtbares Material einschließt.

11. Gestentete Klappe nach einem der vorstehenden Ansprüche, wobei die Drähte der Stentstruktur (10, 20, 30, 40) aus einem Formgedächtnismaterial wie einer Nickel-Titan-Legierung, z. B. Nitinol, ausgebildet sind.

12. Gestentete Klappe nach einem der vorstehenden Ansprüche, wobei die Segel Perikardsegel sind.

13. Abgabesystem, umfassend:
die gestentete Klappe nach Anspruch 8 und
einen Katheter mit einer zurückziehbaren Hülle, die die Stentstruktur (10, 20, 30, 40) abdeckt, bis sie eingesetzt werden soll, wobei die Hülle zurückgezogen werden kann, um der Stentstruktur (10, 20, 30, 40) zu ermöglichen, sich zu expandieren.

## Revendications

1. Valvule à endoprothèse comprenant :
une structure d'endoprothèse (10, 20, 30, 40) comprenant une partie de corps généralement tubulaire ayant une première extrémité (12, 22) et une seconde extrémité (14, 24),
et une structure de valvule fixée à l'intérieur de la partie de corps généralement tubulaire, dans laquelle la structure de valvule comprend des feuillets,
dans laquelle la première extrémité (14, 24) de la structure d'endoprothèse (10, 20, 30, 40) est une extrémité d'entrée et la seconde extrémité (12, 22) de la structure d'endoprothèse (10, 20, 30, 40) est une extrémité de sortie, et dans laquelle la structure d'endoprothèse (10, 20, 30, 40) comprend un certain nombre d'entretoises ou de parties de fils, dans laquelle l'agencement des fils est de telle sorte que la structure d'endoprothèse comprend douze couronnes au niveau de l'extrémité d'entrée de la structure d'endoprothèse (10, 20, 30, 40) et moins de couronnes au niveau de l'extrémité de sortie de la structure d'endoprothèse (10, 20, 30, 40),
dans laquelle la structure d'endoprothèse (10, 20, 30, 40) comporte en outre trois montants longitudinaux (16, 32) pour la fixation de la structure de valvule, dans laquelle les trois montants longitudinaux (16, 32) pour la fixation de la structure de valvule sont constitués de deux barres ou parties verticales qui sont espacées l'une de l'autre d'une distance suffisante pour permettre aux feuillets d'être tirés entre les parties verticales au niveau des commissures des feuillets, et
dans laquelle la structure d'endoprothèse (10, 20, 30, 40) comporte une structure de fil qui s'étend entre une extrémité de chaque montant (16, 32) et la première extrémité (14, 24) de la structure d'endoprothèse (10, 20, 30, 40) et une structure de fil supplémentaire qui s'étend entre l'autre extrémité de chaque montant (16, 32) et la seconde extrémité (12, 22) de la structure d'endoprothèse (10, 20, 30, 40).

2. Valvule à endoprothèse selon l'une quelconque des revendications précédentes, dans laquelle les montants (16, 32) pour la fixation sont plus proches d'un axe central de la structure d'endoprothèse que d'autres éléments d'endoprothèse.

3. Valvule à endoprothèse selon l'une quelconque des revendications précédentes, dans laquelle la structure d'endoprothèse comporte une région centrale (34) dont le diamètre est supérieur à celui des régions adjacentes de la structure d'endoprothèse (10, 20, 30, 40), et dans laquelle les montants (16, 32) pour la fixation sont plus proches d'un axe central de la structure d'endoprothèse que d'autres éléments d'endoprothèse situés dans la région centrale (34) de la structure d'endoprothèse (10, 20, 30, 40).

4. Valvule à endoprothèse selon l'une quelconque des revendications précédentes, dans laquelle une partie centrale de la structure d'endoprothèse (10, 20, 30, 40) est pourvue d'une partie de fil de densité inférieure.

5. Valvule à endoprothèse selon la revendication 4, dans laquelle la valvule à endoprothèse est conçue pour être positionnée en remplacement d'une valvule aortique, et dans laquelle la partie de fil de densité inférieure est pourvue dans la zone de la structure d'endoprothèse (10, 20, 30, 40) positionnée là où les artères coronaires sont situées lorsque la structure d'endoprothèse (10, 20, 30, 40) est implantée.

6. Valvule à endoprothèse selon l'une quelconque des revendications 4 ou 5, dans laquelle la partie de fil de densité inférieure s'étend autour du périmètre entier de la structure d'endoprothèse (10, 20, 30, 40).

7. Valvule à endoprothèse selon l'une quelconque des revendications 4 à 6, dans laquelle les fils de la partie de fil de densité inférieure sont agencés pour fournir des ouvertures qui sont plus grandes que les espaces entre d'autres fils de la structure d'endoprothèse (10, 20, 30, 40).

8. Valvule à endoprothèse selon l'une quelconque des revendications précédentes, dans laquelle la structure d'endoprothèse (10, 20, 30, 40) est compressible pour l'administration percutanée et expansible par l'élimination des forces de compression externes.

9. Valvule à endoprothèse selon l'une quelconque des revendications précédentes, dans laquelle la structure d'endoprothèse (10, 20, 30, 40) est une endoprothèse autoexpansible.

10. Valvule à endoprothèse selon l'une quelconque des revendications précédentes, dans laquelle la structure d'endoprothèse (10, 20, 30, 40) comporte un matériau radio-opaque, un matériau échogène ou un matériau visible à l'IRM.

11. Valvule à endoprothèse selon l'une quelconque des revendications précédentes, dans laquelle les fils de la structure d'endoprothèse (10, 20, 30, 40) sont formés à partir d'un matériau à mémoire de forme tel qu'un alliage de nickel et de titane, par exemple du Nitinol.

12. Valvule à endoprothèse selon l'une quelconque des revendications précédentes, dans laquelle les feuillets sont des feuillets péricardiques.

13. Système d'administration comprenant :
la valvule à endoprothèse selon la revendication 8, et
un cathéter avec une gaine rétractable qui couvre la structure d'endoprothèse (10, 20, 30, 40) jusqu'à ce qu'elle soit déployée, dans lequel la gaine peut être rétractée pour permettre à la structure d'endoprothèse (10, 20, 30, 40) de se déployer.
